# EUROPEAN PATENT APPLICATION

(11) **EP 2 042 172 A1**
(43) Date of publication of application: **01.04.2009**
(21) Application number: 07301397.1
(22) Date of filing: 26.09.2007
(51) Int. Cl.: A61K 31/355, A61P 25/28, A61P 29/00, A61K 31/353

(54) **Use of tocopherol derivatives as inhibitors of the notch signalling pathway**

(71) Applicant: Inserm, 75654 Paris Cedex 13 (FR)
(72) Inventor: Baron-van- Evercooren, Anne, 75005 Paris (FR); Nait Oumesmar, Brahim, 75013 Paris (FR)
(74) Representative: Cabinet Plasseraud

(57) **Abstract**

The invention relates to the use of tocopherol derivatives as inhibitors of the Notch signalling pathway. More particularly, the invention relates to the use of tocopherol derivatives for the treatment of a disease associated with an up-regulated Notch signalling pathway activity.

## Description

### FIELD OF THE INVENTION :

The invention relates to methods for inhibiting the Notch signalling pathway. More particularly, the invention relates to the use of Notch inhibitors for the treatment of a disease associated with an up-regulated Notch signalling pathway activity.

### BACKGROUND OF THE INVENTION :

Notch signalling pathway has a simple framework that is highly conserved throughout the animal kingdom (Artavanis-Tsakonas et al., 1999; Schweisguth, F., 2004; Radtke et al. 2003).

Both the Notch receptor and its ligands, Delta and Serrate (known as Jagged in mammals), are transmembrane proteins with large extracellular domains that consist primarily of epidermal growth factor (EGF)-like repeats. Ligand binding promotes two proteolytic cleavage events in the Notch receptor. The first cleavage is catalysed by ADAM-family metalloproteases, whereas the second is mediated by gamma-secretase, an enzyme complex that contains presenilin, nicastrin, PEN2 and APH1 (Fortini et al., 2002). The second cleavage releases the Notch intracellular domain (Nicd), which then translocates to the nucleus and cooperates with the DNA-binding protein CSL (named after CBF1, Su(H) and LAG-1) and its co-activator Mastermind (Mam) to promote transcription.

The precise numbers of Notch paralogues differ between species. For example, there are four Notch receptors in mammals (Notch1 to 4), two in Caenorhabditis elegans (LIN-12 and GLP-1) and one in Drosophila melanogaster (Notch), but the basic paradigm is common throughout evolution (Artavanis et al., 1999; Schweisguth, 2004; Radtke et al., 2003).

Inhibition of Notch signalling pathway represents a promising tool for treating different kinds of diseases and disorders, such as Alzheimer disease (Beher and Graham, 2005); multiple sclerosis (John et 2002; Nat. Med. 8:1115-1121); brain tumours (Miele et al., 2006), and autoimmune disorders (Briend E, . 2005).

For example, Notch ligand expression plays a role in cancer. Indeed, up regulated Notch ligand expression has been observed in some tumour cells. By downregulating Notch signalling pathway in vivo in T cells, it is possible to prevent tumour cells from inducing immunotolerance in those T cells that recognise tumour-specific antigens. In turn, this allows the T cells to mount an immune response against the tumour cells (see document WO01/35990).

Document WO2006052128 discloses the fact that inhibitors of Notch pathway activation (for example gamma-secretase inhibitors) are extremely useful in the treatment of intestinal adenoma and/or adenocarcinoma

Document W003102242 describes the use of Notch inhibitors for inducing apoptosis for the prevention or treatment of diseases and conditions associated with insufficient apoptosis.

Jurynczyk et al. (2005) show that inhibition of Notch signalling in a demyelinating disease model leads to enhancement of lesion repair with the central nervous system and clinical improvement. These data are supported by recent findings showing the re-expression of the Notch1 receptor ligand, Jagged 1, on the surface of reactive astrocytes in chronic multiple sclerosis lesions. High expresssion of Jagged1 activates Notch signalling in oligodendrocyte precursors, and prevents their differentiation into remyelinating oligodendrocytes (John et al., 2002; Nat. Med.).

### SUMMARY OF THE INVENTION

The invention relates to the use of a compound of formula (I) for inhibiting the Notch signalling pathway 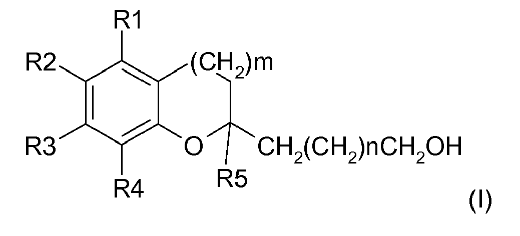 wherein
- R1, R2, R3 and R4 are identical or not, and are each independently a hydrogen, a hydroxyl, a C1-C6 alkyl, a C1-C6 alkoxy or a C1-C6 alkanoyloxy,
- R5 is a hydrogen or a C1-C6 alkyl,
- m is an integer from 0 to 2, preferably 1 or 2, even more preferably 1
- n is an integer from 8 to 25, preferably from 8 to 20, even more preferably between 8 and 16 or between 8 and 14. It encompasses therapeutic uses and non-therapeutic uses.
   The invention also relates a compound of formula (I) for the treatment of a disease associated with an up-regulated Notch signalling pathway activity.
   The invention also relates to the use of a compound of formula (I) for the manufacture of a medicament for the treatment of a disease associated with an up-regulated Notch signalling pathway activity.
   The invention also relates to a method of treatment of a disease associated with an up-regulated Notch signalling pathway activity, said method comprising administrating a compound of formula (I) to a patient in need thereof.
   Another object of the invention is the non therapeutic use of compound of formula (I) for inhibition of the Notch signalling pathway.
   Another object of the invention is a non therapeutic method for inhibiting the Notch signalling pathway comprising the step of adding compound of formula (I) to a cell culture.

### DETAILED DESCRIPTION OF THE INVENTION

### Definitions:

The general chemical terms used throughout have their usual meanings. For example, the term alkyl refers to a branched or unbranched saturated hydrocarbon group. By way of illustration, but without limitation, the term "C1-C6 alkyl" refers to a straight chain or branched hydrocarbon moiety having from 1 to 6 carbon atoms, including methyl, ethyl, propyl, isopropyl, butyl, isobutyl, sec-butyl, tert- butyl, pentyl, neopentyl, n-hexyl...

The term "alkoxy" refers to an alkyl group optionally substituted that is bonded through an oxygen atom. By way of illustration, but without limitation, the term "C1-C6 alkoxy" includes the groups methoxy, ethoxy, isopopoxy, ter-butoxy...

The term "alkanoyloxy" refers to -OCO-alkyl groups, where the term alkyl is as defined above. By way of illustration, but without limitation,the term "C1-C6 alkanoyloxy" , includes acetate, propionate, butylate, pentanoate or hexanoate.

The term "pharmaceutically-acceptable salts" embraces salts commonly used to form alkali metal salts and to form addition salts of free acids or free bases. The nature of the salt is not critical, provided that it is pharmaceutically acceptable. "Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The term "Notch signalling pathway" refers to the biological activity associated with activation or inhibition of the Notch signalling pathway, including any of the downstream biological effects otherwise resulting from the binding Notch receptors of its natural ligand.

The term "disease associated with an up-regulated Notch signalling pathway activity" includes any disease which results from the activation or the up-regulated activity of the Notch signalling pathway or is associated with the activation or the up-regulated activity of the Notch signalling pathway. Assays for the activation of the up-regulated activity of the Notch signalling pathway can include the quantification of the expression of the genes downstream of Notch, such as Hes1 and Hes5, eg by quantitative RT-PCR, northern-blot and immunological assays In the context of the invention, the term "treating" or "treatment", as used herein, means reversing, alleviating, inhibiting the progress of, or preventing the disorder or condition to which such term applies, or one or more symptoms of such disorder or condition. "Therapeutically effective amount" means an amount of a compound/ medicament according to the present invention effective in producing the desired therapeutic effect.

According to the invention, the term "patient", or "patient in need thereof", is intended for a human or non-human mammal affected or likely to be affected with a disorder. Preferably, the patient is a human.

### Therapeutic compounds, methods and uses:

The present invention provides the first demonstration that compounds of formula (I) are Notch signalling pathway inhibitors.

Therefore, the present invention relates to compounds of formula (I) for the treatment of a disease associated with an up-regulated Notch signalling pathway activity. 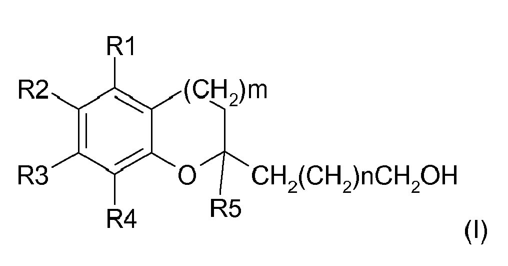 wherein
- R1, R2, R3 and R4 are identical or not, and are each independently a hydrogen, a hydroxyl, a C1-C6 alkyl, a C1-C6 alkoxy or a C1-C6 alkylcarbonyl,
- R5 is a hydrogen or a C1-C6 alkyl,
- m is an integer from 0 to 2, preferably 1 or 2, even more preferably 1
- n is an integer from 8 to 25, preferably from 8 to 20, even more preferably between 8 and 16 or between 8 and 14.

The compounds of the present invention contain an asymmetric carbon atom (the one bearing R5), and, therefore, the instant invention may also include the individual diastereomers and enantiomers, which may be prepared or isolated by methods known to those skilled in the art. It will be appreciated that the compounds of the invention may be isolated in optically active or racemic forms. Thus, all chiral, diastereomeric, racemic forms and all geometric isomeric forms of a structure are intended, unless the specific stereochemistry or isomeric form is specifically indicated. It is well-known in the art how to prepare and isolate such optically active forms. For example, mixtures of stereomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

The compounds of formula (I) and methods for preparing them are described in the International Patent Application n° WO2005/030748 which is incorporated herein by reference.

In a particular embodiment, m is 1 and the compounds are named Tocopherol Fatty Alcohols (TFA-n), where n is defined as above.

Compounds for which n is 10, 11, 12, 13, 14, 15 or 16 are particularly suitable for the invention.

In another particular embodiment, preferred compounds are selected from the group consisting of TFA-12, TFA-14, TFA-15, TFA-16 and TFA-18.

In another embodiment, the preferred compound is TFA-12 which has the formula:

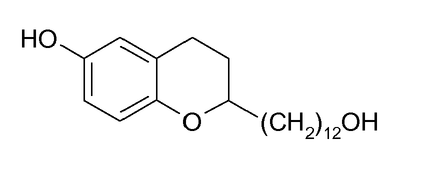

Diseases associated with an up-regulated Notch signalling pathway activity include but are not limited to cancer, central nervous system diseases such as multiple sclerosis, Alzheimer and more generally CNS diseases associated with inflammation.

In one embodiment, the disease is cancer. In another embodiment, the disease is multiple sclerosis.

In another embodiment the disease is a disease associated with an up-regulated Notch signalling pathway activity excepting nervous system diseases affecting oligodendrocytes or other cells of the nervous system, inflammation of the nervous system, degenerative neuropathies, demyelinating diseases, multiple sclerosis, Alzheimer's disease, Parkinson's disease, Creutfeldt-Jakob's disease, strokes or any other lesion of the nervous system.

Another object of the invention relates to a method for treating a disease associated with an up-regulated Notch signalling pathway activity comprising administering to a patient in need thereof a therapeutically effective amount of a compound of formula (I).

It will be understood that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

### Pharmaceutical compositions:

The compound of formula (I) may be combined with pharmaceutically acceptable excipients, and optionally sustained-release matrices, such as biodegradable polymers, to form therapeutic compositions.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The compounds of formula (I) can be formulated into a composition in a neutral or salt form. Suitable pharmaceutically-acceptable acid addition salts of compounds of Formula I may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, example of which are formic, acetic, propanoic, succinic, glycolic, gluconic, lactic, malic, tartaric, citric, ascorbic, glucuronic, maleic, fumaric, pyruvic, aspartic, glutamic, benzoic, anthranilic, mesylic, salicyclic, salicyclic, p- hydroxybenzoic, phenylacetic, mandelic, embonic (pamoic) , methanesulfonic, ethanesulfonic, benzenesulfonic, pantothenic, 2-hydroxyethanesulfonic, toluenesulfonic, sulfanilic, cyclohexylaminosulfonic, stearic, algenic, β- hydroxybutyric, salicyclic, galactaric and galacturonic acid. Suitable pharmaceutically-acceptable base addition salts of compounds of formula I include metallic salts made from aluminum, calcium, lithium, magnesium, potassium, sodium and zinc or organic salts made from N,N'- dibenzylethylenediamine, chloroprocaine, choline, diethanolamine, ethylenediamine, meglumine (N- methylglucamine) and procaine. All of these salts may be prepared by conventional means from the corresponding compound of formula I by reacting, for example, the appropriate acid or base with the compound of formula I.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The compounds of formula (I) may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of formula (I) formulated for parenteral administration, such as intravenous or intramuscular injection, other pharmaceutically acceptable forms include, e.g. tablets or other solids for oral administration ; liposomal formulations ; time release capsules ; and any other form currently used.

### Non therapeutic compounds, methods and uses:

The invention also relates to compounds of formula (I) for non-therapeutical use and methods comprising administering compounds of formula (I) to cell cultures.

The invention will further be illustrated in view of the following figures and examples.

### FIGURE LEGENDS

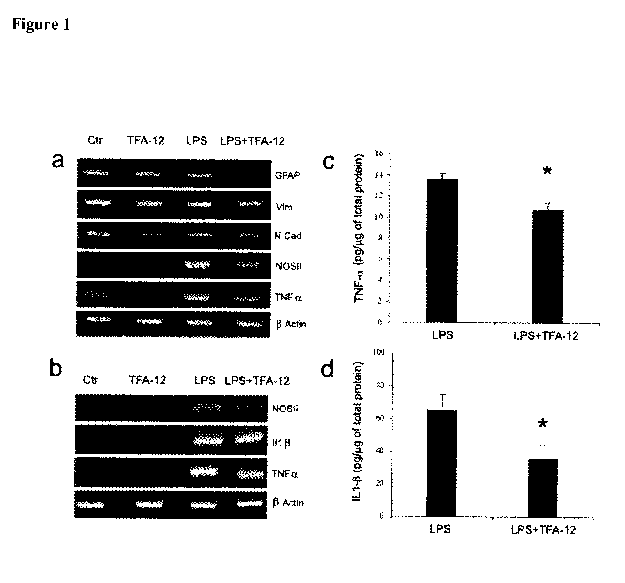

### Fig. 1: TFA-12 inhibits astroglial and microglial activation in vitro.

(a): RT-PCR analysis of GFAP, vimentin, N-Cadherin, NOSII and TNF-α expression in astrocyte primary cultures, treated or untreated with TFA-12 (10⁻⁶ M), and with or without LPS (1 µg/ml). Cells were incubated with TFA-12 and LPS for 24 hours and RNA extraction was performed using Trizol reagent. TFA-12 inhibits both astrogliosis and inflammatory gene response. **(b):** RT-PCR gene expression analysis in MMGT12 microglial cell line. RNA extraction was performed after 6 hours of incubation with TFA-12 (10⁻⁵ M), with or without LPS activation (0.01 µg/ml). Expression of NOS-II, IL-1β and TNF-α transcripts in TFA-12- treated MMGT12 cells are significantly decreased. β-actin was used as an internal control. **(c,d):** ELISA quantification of TNFα **(c)** and IL1β **(d)** secretion in LPS-stimulated microglial cell line cultures, treated and untreated with TFA-12 during 24 hours. TFA-12 inhibits both TNF-α and IL-1β secretion. Data are mean ± SEM values obtained from three experiments, each conducted in independent cultures (n = 3). **p*<0.01.

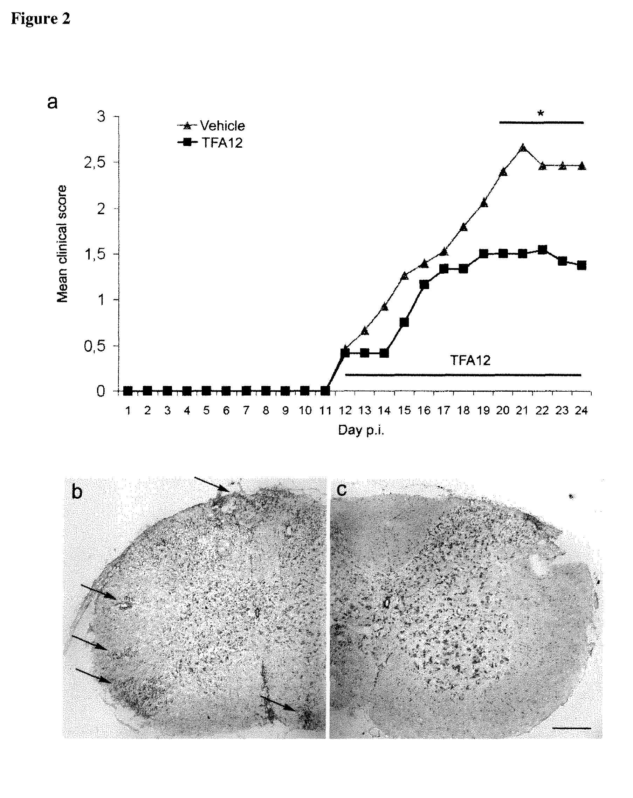

### Fig. 2: TFA-12 improves the clinical course of the EAE disease.

**(a):** EAE was induced in C57BL/6 mice by immunization with MOG₃₅₋₅₅ peptides (*n*=40) and clinical scores were evaluated daily. TFA-12 (0.39 mg/kg in NaCl) or saline solution alone was injected daily after disease onset (day 12 post-immunization). **(a) :** TFA-12 injections improves the mean clinical scores in EAE mice compared with vehicle treatment (*n*=20 for each group). From 20 to 24 days post-immunization, clinical scores were significantly lower in TFA-12-injected mice compared to controls (**p*<0.05), **(b,c) :** LFB-Cresyl violet staining of vehicle-treated **(b)** and TFA-12-treated **(c)** spinal cord sections, 24 days post-immunization. Vehicle-treated mice present large inflammatory foci (arrows) in the spinal cord white matter **(b),** whereas no inflammation is detected in TFA-12-treated mouse spinal cord **(c).** Scale bar, 100 µm.

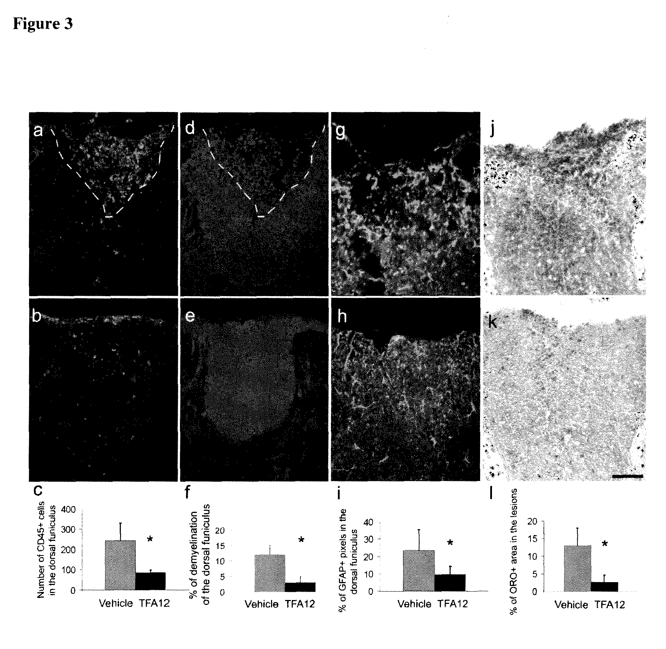

### Fig.3: Inflammation, astrogliosis and demyelination are reduced by TFA-12 treatment.

**(a,b,c) :** CD45 immunohistochemistry on spinal cord sections of vehicle- **(a)** and TFA-12- treated **(b)** mice reveals immune foci (dashed lines) in the dorsal funiculus. **(c):** The number of CD45+ cells is decreased by 2.8 fold in TFA-12-treated mice compared to controls. **(d,e,f) :** In vehicle treated mice, demyelinated lesions are characterised by the absence of MBP staining **(d),** whereas demyelination is barely visible in TFA-12-treated groups. The extent of the demyelination in the spinal cord is 4.2 fold lower in the TFA-12-treated group than the vehicle-treated one **(f). (g,h,i):** Astrogliosis is also drastically reduced in TFA-12-treated animals, as shown by GFAP staining in the spinal cord of vehicle treated **(g)** and TFA-12-treated mice **(h).** The percentage of GFAP immunoreactivity is reduced by 2.4 fold in the TFA-12 group **(i). (j,k,l) :** In vehicle-treated mice, ORO staining (red) indicates that most of lesions remains active with an intense macrophage activity **(j),** whereas in TFA-12-treated mice, macrophage activity is reduced in the lesions **(k).** The percentage of ORO+ demyelinated area is reduced by 5.5 fold in TFA-12-treated mice **(I).** All quantifications were made on 3 independent experiments. **p*<0.05, scale bar, 50 µm.

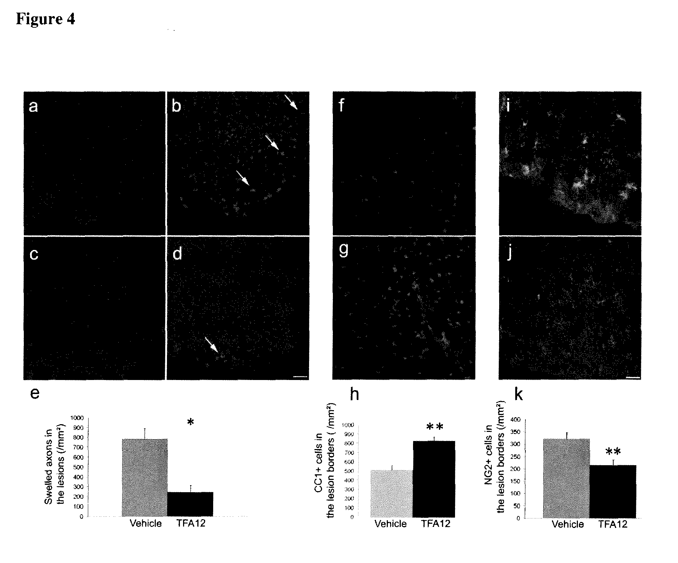

### Fig.4: TFA-12 induces oligodendrocyte differentiation and reduces axonal damage in EAE.

**(a-d):** Spinal coronal sections stained with the 2H3 anti-neurofilament antibody **(b,d)** and counterstained with the nuclear dye Hoechst 33342 **(a,c).** Intense axonal swelling (arrows) is detected in white matter lesions of vehicle-treated spinal cords **(b),** while very few degenerating axons are evidenced in TFA-12-treated spinal cord lesions **(d). (e):** Axonal degeneration is 3.3 fold lower in TFA-12-treated mice than controls. **(f,g):** CC1+ oligodendrocytes are more abundant at the periphery of spinal cord lesions in TFA-12-treated mice **(g)** compared to controls **(f). (h):** Quantification of CC1+ cells in the lesion borders shows a 1.6 fold increase of differentiated oligodendrocytes in the TFA-12-treated group. **(i,j)** OPCs stained for NG2 in the periphery of the lesions, in controls (i) and TFA-12-treated mouse spinal cords **(j). (k):** TFA-12 treatment reduces by 1.5 fold the number of NG2+ cells in the lesion borders. **p*<0.05; ***p*<0.01, scale bar, 50 µm.

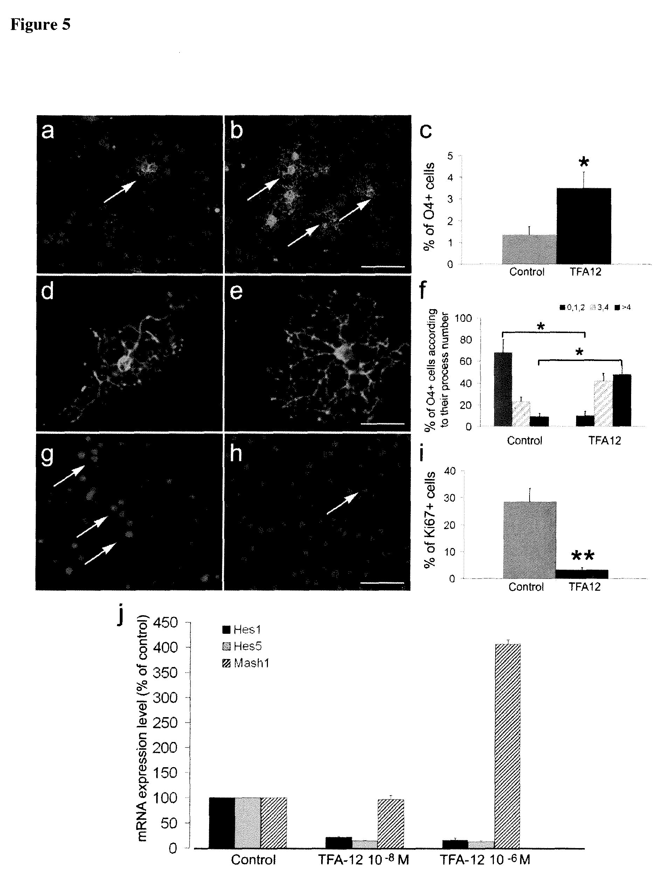

### Fig 5: TFA-12 promotes OPC differentiation in vitro.

The CG4 oligodendroglial cells were differentiated in N1 medium **(a, d, g)** or in N1 supplemented with 5.10⁻⁷ M of TFA-12 **(b, e, h),** during 48 hours. The number of 04+ oligodendrocytes (arrows) is increased by TFA-12 treatment **(b)** compared to untreated cultures **(a). (c):** The percentage of O4+ oligodendrocytes is 2.5 fold higher in TFA-12-treated than untreated cultures. **(d, e):** Morphology of untreated **(d)** and TFA-12-treated **(e)** 04+ oligodendrocytes. **(f):** Quantification of 04+ oligodendrocytes, according to their process number, shows that TFA-12 increases the number of processes emerging from the cell body, thus confirming that this compound promotes OPC differentiation. Proliferating Ki67+ cells (arrows) are reduced in N1 medium plus TFA-12 **(h)** compared to N1 alone **(g). (i):** Quantification of the percentage of Ki67+ cells shows a 11 fold reduction of cell proliferation. **(j):** Quantitative RT-PCR of Hes1, Hes5 and Mash1 gene expression levels in CG4 cells untreated and treated with TFA-12. The expression of Notch downstream effectors, Hes1 and Hes5 is reduced by 4 fold by TFA-12 treatment, while Mash1 expression is increased by 4 fold, indicating that this compound acts as potent inhibitor of the Notch signalling. Wilcockson test performed on ΔCt was used to confirm significance between TFA-12 and control groups (*p*<0.05). Student *t* test: **p*<0.05, ***p*<0.001. Scale bars, 50 µm (a,b,g,h); 25 µm (d, e).

### EXAMPLE:

### METHODS

### Cell cultures

The CG4 oligodendrocyte precursor cell line was cultured on polyornithine-laminine and expanded in N1/B104 (70v/30v) medium, as previously described (Louis et al., 1992). For differentiation experiments, N1/B104 was replaced by N1 containing TFA-12 at 5.10⁻⁷ M or by N1 alone. Media were changed daily and differentiation was carried out during 48 hours. Primary astrocyte cultures were prepared from newborn Wistar rat pups, as previously reported (Morga et al., 2000). The murine microglial cell line MMGT12 (gift from Dr. Vanmechelen, Innogenetics, Gent, Belgium) was cultured in DMEM/Ham's F12 (1/1), supplemented with 2% fetal calf serum (FCS, Invitrogen), 1% Insulin/Transferrin/Selenium (ITS, Invitrogen) and 15% filtered conditioned medium of WEHI cells (WEHI3, WEHI 3B and WEHI 3D, ATCC). This cell line presents a behavior similar to that of primary microglia (Briers et al., 1994; Balboa et al., 2001; Hemmer et al., 2001). For astrocyte cultures, TFA-12 treatment (10⁻⁶ M) was performed during 24 hours, with or without LPS (1 µg/ml). MMGT12 cultures were treated with TFA-12 (10⁻⁵ M) with or without LPS stimulation (0.01 µg/ml), during 6 hours for RT-PCR and 24 hours for ELISA experiments. The synthesis of the tocopherol long chain fatty alcohol TFA-12 was performed as previously described (Muller et al., 2004; 2006).

### EAE induction and TFA-12 treatment

Twelve weeks-old C57BL/6 female mice (Elevage Janvier) were immunized sub-cuteanously with an emulsion consisting of 200 µg of synthetic MOG₃₅₋₅₅ peptides (NeoMPS) in complete Freund's adjuvant (CFA, Difco), supplemented with 500 µg of heat-inactivated *Mycobacterium tuberculosis* (strain H37Ra, Difco). Mice were injected intravenously with Pertussis toxin (List Biological Laboratories) on the day of the immunization and 48 hours later, and then divided in two groups. Animals were scored daily for clinical signs (0 = healthy; 1 = loss of tail tone; 2 = hindlimb weakness; 3 = hindlimb paralysis; 4 = scale 3 plus forelimb weakness; 5 = death). From day 12 post-immunization until the end of experiment, one group (n=20) was injected daily with TFA-12 (i.p injection, 0.39 mg/kg in 1% ethanol/saline solution) and the other group (n=20) with the vehicle solution only. Animals were euthanized at 25 days post-immunization. All experiments were performed according to European Community regulations and ethical policies and approved by INSERM ethical committee (authorization 75-348; 20/04/2005).

### Tissue Processing

Animals were perfused under deep anaesthesia with a 4% paraformaldehyde (PFA) solution in 0.1M PBS, pH7.4. Spinal cords were removed, post-fixed in the same fixative at 4°C for 2 hours, and incubated overnight in a solution of 15% sucrose in 0.1M PBS at 4°C. Tissues were frozen in isopentane (-60°C) and 12-µm coronal serial sections were collected on Superfrost-plus slides, using a Reichert cryotome (Leica).

### Histological staining

Sections were stained with either Luxol fast blue (LFB; to visualize myelin, from Sigma) or Oil Red O (ORO; to visualize macrophages containing myelin debris, from Sigma). For LFB staining, sections were dehydrated and then incubated in a 0.1% LFB solution at 37°C overnight. Slides were then cooled at 4°C, incubated in a 0.05% lithium carbonate solution, and cleared in 70% ethanol. Slides were next stained with Cresyl violet, rinsed twice, dehydrated and mounted in Eukitt (O.Kindler Gmbh). For ORO staining, sections were incubated in a 0.15% ORO/36% triethyl phosphate solution, rinsed in water, incubated with hematoxylin for nuclear staining and mounted in Fluoromount medium (Clinisciences).

### Immunohistochemistry

Sections were pre-incubated in 90% ethanol solution (5 min) for MOG and MBP staining. They were incubated overnight with primary antisera at 4°C, rinsed, and incubated with the appropriate secondary antibodies for 1 hour at 20°C. Sections were counterstained with bisbenzimide (Hoescht 33342, Sigma), washed, and mounted with Fluoromount (Clinisciences). For *in vitro* experiments, cells were incubated with 04 primary antibody (1:1; ATCC) for 30 min at 20°C. For Ki67 staining (1:100; BD Pharmingen), cells were fixed with ice cold 2% PFA, rinsed twice with PBS, and then incubated with the primary antibody. Cells were incubated with secondary antibodies for 30 min, rinsed in PBS, counterstained with Hoescht 33342, and mounted with fluoromount (Clinisciences). Slides and cells were analyzed under a fluorescence microscope (DMRA, Leica). For *in vivo* experiments, the following antibodies were used : rabbit anti-MBP (1:100; Chemicon) and mouse anti-MOG (1:20; 8-18C5 hybridoma, gift from C. Linington, Department of Medicine and Therapeutics, University of Aberdeen, U.K) for myelin staining ; rat anti-CD45 (1:100; Caltag) for immune cells; rabbit anti-NG2 (1:100; Abcys) for OPCs; mouse anti-APC7 (1 :100 ; clone CC1, Invitrogen) for postmitotic oligodendrocytes;mouse monoclonal anti-neurofilament 165 kDa (1:2; 2H3 antibody from the developmental studies hybridoma bank, University of Iowa). **Morphological quantification**

*In vitro,* cell counts were established on 3 separates experiments. Quantifications are mean ± SEM values of the data collected from at least 3 coverslips for each experiment. In order to avoid subjective selection, microscopic fields were randomly selected and at least 500 cells were counted for each coverslip. For *in vivo* experiments, cell counts were established on a minimum of 3 animals. For each animal, staining and cell counts were made on at least 3 slides containing 15 sections of 12 µm thickness and covering the cervical, thoracic and lumbar levels of the spinal cord. To limit experimental bias, all slides were stained at the same time and all images were taken with the same acquisition parameters. Measurements were performed in the lesions, on images taken at a magnification of 40X and processed with ImageJ 1.34S, except for NG2 and CC1 immuno-positive cells, which were counted in the margins of the lesions (area comprised between the limits of the lesion and 200 µm apart). Lesions were identified by the presence of inflammatory foci and by the lack of MBP immunolabelling.

### Real-time PCR

Total RNA was isolated using Trizol reagent (Invitrogen). DNA contamination was removed with DNasel treatment and then 1 µg of RNA was synthesized into first strand cDNA using Thermoscript RT-PCR System kit (Invitrogen). Three replicates of real-time PCR experiments were performed using an ABlprism 7000 sequence detection system (Applied Biosystems) with Taqman gene expression assay probes (Applied Biosystems). After real-time PCR experiment Ct number was extracted for both reference gene (GAPDH) and target gene with auto baseline and manual threshold. We used the ΔΔCt model to determine fold increases and Wilcockson test on ΔCt for statistical analysis.

### Statistical analysis

Student *t*-test and Mann-and-Whitney *U* test were used to assess statistical significance (SigmaStat 3.1, Systat Software Inc.). *P* values < 0.05 were considered significant.

### RESULTS

### TFA-12 inhibits astroglial and microglial activation in vitro

As a first screen of the biological activity of TFA-12, we tested whether this molecule could modulate astroglial and microglial activation induced by LPS treatment (1µg/ml and 0.01 µg/ml for astroglial and microglial cultures, respectively). In primary astrocyte cultures, PCR experiments for GFAP, vimentin and N-Cadherin indicated that TFA-12 reduces the expression of astroglial genes, even in response to LPS activation **(****Fig. 1a****).** NOSII and TNFαgene expression were also significantly downregulated in astrocyte cultures treated with TFA-12. The same experiment was performed on primary microglial cell line (MMGT12 cells), which were induced to produce TNF-α, IL-1β and NOSII transcripts, in response to LPS stimulation during 24 hours. Addition of TFA-12 to LPS-treated cultures effectively inhibits the expression of major mediators of microglial activation such as TNF-α and NOSII **(****Fig. 1b****).** This inhibition was further confirmed by ELISA analysis of TNF-α **(****Fig. 1c****)** and IL-1β **(****Fig. 1d****)** protein levels in TFA-12-treated culture supernatants. These data demonstrate that TFA-12 is an effective inhibitor of astroglial and microglial activation, and therefore may prevent inflammatory insults in the CNS.

### TFA-12 enhances clinical recovery in experimental autoimmune encephalomyelitis (EAE)

These studies prompted us to test whether TFA-12 could have therapeutic effects on the clinical course of MOG-induced EAE in mouse, a chronic inflammatory demyelinating model that mimics important pathological hallmarks of MS lesions. In this model, inflammation and demyelination occur mainly in the spinal cord, leading to chronic lesions with axonal loss (Hobom et al., 2004). EAE-induced mice were injected daily with TFA-12 (i.p injection, 0.39 mg/kg, n= 20) or the vehicle alone (saline solution with 0.2% alcohol, n= 20) from the onset of clinical signs (day 12 post-immunisation) to the end of the experiment (day 24 post-immunization). Mice treated with the vehicle alone showed a clinical course typical of MOG-induced EAE **(****Fig. 2a****).** Vehicle-treated EAE mice presented clinical scores increasing from day 12 to day 21 post immunization, reaching a mean clinical score of 2.5. In contrast, TFA-12-treated mice displayed a significant reduction of the EAE disease, as assessed by the mean clinical score which never reached a score greater than 1.5. Differences in clinical recovery between vehicle- and TFA-12-treated groups became significant 21 days post-immunization (*p*<0.05) and were reproduced in 3 independent EAE experiments. To validate at the histological level, the clinical improvement induced by TFA-12, we stained coronal spinal cord sections of TFA-12- and vehicle-treated animals with Luxol Fast blue/Cresyl violet to verify myelin integrity and cellular infiltrates, respectively. This staining revealed numerous inflammatory foci in the vehicle-treated mouse spinal cord **(****Fig. 2b****),** which were predominant in the dorsal funiculus and ventral white matter tracts. In contrast, TFA-12- treated mice showed a much lower extent of cellular infiltrates **(****Fig. 2c****),** indicating that this molecule attenuates significantly CNS inflammation.

### TFA-12 reduces inflammation, astrogliosis and demyelination in EAE disease

To test whether the clinical improvement of EAE-mice treated with TFA-12 may be due to the anti-inflammatory properties of this molecule, we stained spinal cord sections with CD45 to identify leucocytes, 24 days post-immunization. In TFA-12-treated mice, CD45 staining revealed very few immune infiltrates with CD45+ cells often disseminated in the white matter tracts **(****Fig. 3b****).** In EAE mice treated with the vehicle alone, numerous CD45+ cells were detected in the lesioned areas **(****Fig. 3a****),** which were correlated with the lack of MBP immunostaining **(****Fig. 3d****).** We quantified the number of CD45+ cells in the dorsal funiculus of both groups and demonstrated that the number of leucocytes was decreased by 2.8 fold in TFA-12-injected EAE mice compared to controls **(****Fig. 3c****).** These results indicate that TFA-12 acts a potent inhibitor of the CNS inflammation. We next analyzed the demyelination status in the spinal dorsal column, using MBP immunohistochemistry **(****Fig.3 d,e****).** Vehicle- treated mice presented large demyelinated lesions filled with CD45+ cells **(****Fig. 3a****).** These lesions were characterized by the absence of a homogenous MBP staining and the presence of punctuate MBP+ myelin debris **(****Fig. 3d****).** In contrast, TFA-12-treated mice showed much smaller demyelinated lesions **(****Fig. 3e****)** compared to controls. Quantification of the demyelinated lesions, determined as the percentage of MBP-negative areas in the dorsal funiculus, revealed that TFA-12 reduced the extent of demyelination by 4.2 fold **(****Fig. 3f****).** As stated earlier, TFA-12 inhibits both astroglial and microglial activation *in vitro.* To test whether such effect could be observed *in vivo* after EAE induction, we stained spinal cords of both groups for astrocytes and activated microglial cells, using immunohistochemistry for GFAP and F4/80 respectively. We showed that astrocytes expressed lower levels of GFAP in spinal cord lesions of TFA-12-treated mice compared to controls **(****Fig. 3g,h****).** Quantification of GFAP immunostaining revealed a 2.4 fold reduction of the reactive astrogliosis, after TFA-12 treatment **(****Fig. 3i****).** Microglial activation was also strongly attenuated in TFA-12-treated mouse spinal cords, as compared to controls. To confirm these observations, we stained spinal cord sections with Oil Red-O (ORO) that specifically revealed myelin debris in macrophages, a feature of active demyelination. In vehicle treated mice, cellular infiltrates presented intense ORO staining, indicating that demyelination was occurring in the lesions **(****Fig. 3j****).** In contrast, lesions were rarely labelled with ORO in spinal cords of the TFA-12-treated groups **(****Fig. 3k****),** confirming that demyelination was prevented by administration of this tocopherol long chain fatty alcohol. We quantified ORO-labelled macrophage infiltrates and found a 5.5 fold decrease in spinal cords of TFA-12-treated mice compared to controls **(****Fig. 3l****).**

### TFA-12 reduces axonal damage and induces oligodendrocyte differentiation in EAE lesions

Since tCFA15, a related long chain fatty alcohol, was shown to promote axonal growth *in vitro,* even in the presence of inhibitory signals (Hanbali et al., 2004), we next studied the effects of TFA-12 on axonal damage occurring during MOG-induced EAE. The axonal swelling, typical of the Wallerian degeneration (Rovaris et al., 2005), was detected by immunohistochemistry against the mouse anti-neurofilament antibody 2H3. In vehicle-treated animals, swelling axons were frequently observed within and at the periphery of white matter lesions, which also exhibited a substantial axonal loss **(****Fig. 4a,b****).** In TFA-12-treated mice, axonal damage was reduced, as evidenced by the decreased number of swelling elements compared to controls **(****Fig. 4c,d****).** Quantification showed a 3.3 fold reduction of axonal damage in TFA-12-treated mice **(****Fig. 4e****),** indicating that this compound has also a potent neuroprotective effect in EAE. However, we could not determine in this model, if the neuroprotection elicited by TFA-12 is through a direct trophic effect on the axons or secondary to its anti-inflammatory properties. To assess whether TFA-12 could promote remyelination in EAE lesions, we examined the potential effect of this molecule on OPC differentiation, using anti-NG2 and anti-CC1 antibodies. Quantification of immature NG2+/CC1- OPCs and differentiated NG2-/CC1+ oligodendrocytes was performed in the lesion margins. We showed that TFA-12 treatment increased by 1.6 fold the number of mature NG2-/CC1+ oligodendrocytes compared to vehicle-treated mice **(****Fig. 4f,g,h****).** In contrast, the number of NG2+/CC1- OPCs, detected in the periphery of lesions, was decreased by 1.5 fold in the TFA-12-treated group **(****Fig. 4i,j,k****),** attesting that this molecule had a pro-differentiation effect on oligodendroglial cells.

### TFA-12 promotes OPC differentiation through the inhibition of Notch signalling.

To examine whether TFA-12 mediated oligodendrocyte differentiation could be explained by a direct action on OPCs, we analysed the effects of this compound *in vitro* on the CG4 rat OPCs cell line (Louis et al., 1992). The CG4 cells were maintained as immature A2B5+/04- OPCs in N1 medium supplemented with B104 conditioned medium and induced to differentiate into 04+ oligodendrocytes in N1 basal medium without serum (Louis et al., 1992). We assessed the effects of TFA-12 (5.10⁻⁷ M) on CG4 cells in basal medium, using immunocytochemistry for A2B5, a marker of OPCs and 04 which labels pre-oligodendrocytes and mature oligodendrocytes. In these conditions, 48 hours treatment with TFA-12 increased by 2.5 fold the number of 04+ oligodendrocytes compared to control cultures **(****Fig. 5a,b,c****).** Moreover, the number of GaIC+ differentiated oligodendrocytes was also enhanced in TFA-12 experiments (data not shown). We evaluated the degree of differentiation of the CG4 cells in both condition, by counting the total number of 04+ oligodendrocytes with 0 to 2, 3 to 4 and more than 4 processes emerging from the cell body. Under TFA-12 treatment, CG4 cells were significantly more branched compared to vehicle-treated cells **(****Fig. 5d,e,f****),** confirming that this molecule promotes OPC differentiation. As differentiation is often correlated with cell cycle exit, we used Ki67 labelling to quantify proliferation in the CG4 cell cultures. Under TFA-12 treatment, the number of Ki67+ cells was reduced by 11 fold compared to the controls **(****Fig. 5g,h,i****).**

The Notch signalling pathway plays a crucial role in the control of the oligodendrocyte development (Park et al., 2005; Gaiano et al., 2002). Activation of Notch receptors in OPCs was shown to maintain OPCs at a proliferating stage and inhibit their differentiation. Notch activation induces the expression of the downstream effectors Hes1 and Hes5, which down-regulate gene transcription of b-HLH transcription factors such as Mash1. Therefore, we tested whether this compound could modulate Notch signalling in oligodendrocytes. Interestingly, both Hes1 and Hes5 expression, assessed by real time PCR, were decreased by 4 fold in CG4 cultures treated with TFA-12 concentrations between 10⁻⁸ M and 10⁻⁶ M **(****Fig. 5j****).** Inhibition of Hes gene expression in TFA-12-treated cultures was further correlated with a 4 fold increase of Mash1 transcripts (Fig. **5j****),** a b-HLH factor involved in oligodendrocyte specification and differentiation (Paras et al., 2007). Altogether, these results provide compiling evidences indicating that TFA-12 promotes oligodendrocyte differentiation, through the inhibition of Notch signalling.

### Conclusion

Multiple sclerosis (MS) is a chronic inflammatory disease of the central nervous system, leading to demyelination and axonal loss, which results in irreversible impairments of neurological functions. Currently available treatments are mostly targeting the inflammatory component of the disease and none of these therapies enhance myelin and axonal repair. In this study, we tested the effects of the tocopherol long chain fatty alcohol TFA-12 in a mouse model of MOG-induced experimental autoimmune encephalomyelitis (EAE) that shares important pathological hallmarks with MS. We show that daily intra-peritoneal injections of TFA-12 reduce significantly the clinical score and severity of the disease. Histological evaluations indicate that TFA-12 treatment decreases the extent of several deleterious components of EAE, including inflammation, astrogliosis, demyelination and axonal damage. Furthermore, we demonstrate both *in vitro* and *in vivo* that TFA-12 promotes oligodendrocyte precursor differentiation through the inhibition of the Notch pathway. Altogether, these results indicate that TFA-12 has a relevant therapeutic potential for the treatment of diseases associated with an up-regulated Notch signalling pathway activity such as inflammatory demyelinating CNS disorders, MS in particular.

### REFERENCES:

Artavanis-Tsakonas, S., Rand, M. D. & Lake, R. J. Notch signaling: cell fate control and signal integration in development. Science 284, 770-776 (1999).
Back, S.A. et al. Hyaluronan accumulates in demyelinated lesions and inhibits oligodendrocyte progenitor maturation. Nat Med 11, 966-72 (2005).
Beher and Graham, Expert Opinion on Investigational Drugs (2005).
Briend E, Curr Opin Mol Ther. ;7(1):56-61 (2005).
Fortini, M. E. Gamma-secretase-mediated proteolysis in cell-surface-receptor signalling. Nature Rev. Mol. Cell Biol. 3, 673-684 (2002).
Gaiano, N. & Fishell, G. The role of notch in promoting glial and neural stem cell fates. Annu Rev Neurosci 25, 471-90 (2002).
Hanbali, M. et al. Counteraction of axonal growth inhibitory properties of Semaphorin 3A and myelin-associated proteins by a synthetic neurotrophic compound. J Neurochem 90, 1423-31 (2004).
Hobom, M. et al. Mechanisms and time course of neuronal degeneration in experimental autoimmune encephalomyelitis. Brain Pathol 14, 148-57 (2004).
Hu, Q.D. et al. F3/contactin acts as a functional ligand for Notch during oligodendrocyte maturation. Cell 115, 163-75 (2003).
John, G.R. et al. Multiple sclerosis: re-expression of a developmental pathway that restricts oligodendrocyte maturation. Nat Med 8, 1115-21 (2002).
Jurynczyk, M., Jurewicz, A., Bielecki B., Raine, C., Krzystof S. Inhibition of Notch signalling enhances tissue repair in an animal model of multiple sclerosis. J Neuroimmuno. 170, 3-10 (2005).
Kageyama, R. & Ohtsuka, T. The Notch-Hes pathway in mammalian neural development. Cell Res 9, 179-88 (1999).
Louis, J.C., Muir, D. & Varon, S. Autocrine inhibition of mitotic activity in cultured oligodendrocyte-type-2 astrocyte (O-2A) precursor cells. Glia 6, 30-8 (1992).
Miele et al., Curr Cancer Drug Targets. 2006 Jun;6(4):313-323), and autoimmune disorders (2006).
Muller, T., Grandbarbe, L., Morga, E., Heuschling, P. & Luu, B. Tocopherol long chain fatty alcohols decrease the production of TNF-alpha and NO radicals by activated microglial cells. Bioorg Med Chem Lett 14, 6023-6 (2004).
Park, H.C., Boyce, J., Shin, J. & Appel, B. Oligodendrocyte specification in zebrafish requires notch-regulated cyclin-dependent kinase inhibitor function. J Neurosci 25, 6836-44 (2005).
Parras, C.M. et al. The proneural gene Mash1 specifies an early population of telencephalic oligodendrocytes. J Neurosci 27, 4233-42 (2007).
Radtke, F. & Raj, K. The role of Notch in tumorigenesis: oncogene or tumour suppressor? Nature Rev. Cancer 3, 756-767 (2003).
Schweisguth, F. Notch signaling activity. Curr. Biol. 14, R129-R138 (2004).
Wang, D. et al. Astrocyte-associated axonal damage in pre-onset stages of experimental autoimmune encephalomyelitis. Glia 51, 235-40 (2005).

## Claims

1. Use of a compound of formula (I) for inhibiting the Notch signalling pathway 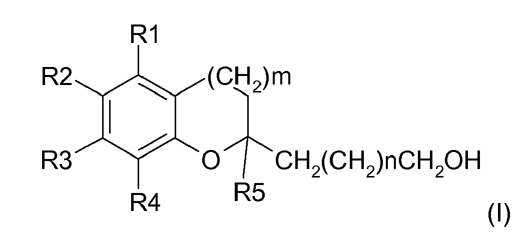 wherein
- R1, R2, R4 and R4 are identical or not, and are each independently a hydrogen, a hydroxyl, a C1-C6 alkyl, a C1-C6 alkoxy or a C1-C6 alkanoyloxy,
- R5 is a hydrogen or a C1-C6 alkyl,
- m is an integer from 0 to 2,
- n is an integer from 8 to 25.

2. A compound of formula (I) for the treatment of a disease associated with an up-regulated Notch signalling pathway activity.

3. Use of a compound of formula (I) for the manufacture of a medicament for the treatment of a disease associated with an up-regulated Notch signalling pathway activity.

4. A method of treatment of a disease associated with an up-regulated Notch signalling pathway activity, said method comprising administrating a compound of formula (I) to a patient in need thereof.

5. A compound according to claim 2 or use according to claim 3 or method according to claim 4 wherein said disease is selected from the group consisting of cancer, central nervous system diseases such as Alzheimer, multiple sclerosis, and CNS diseases associated with inflammation.

6. A compound according to claim 2 or use according to claim 3 or method according to claim 4 wherein said disease is multiple sclerosis.

7. Use according the claim 1, wherein the use is non-therapeutic.

8. Non therapeutic method for inhibiting the Notch signalling pathway comprising the step of adding compound of formula (I) as defined in claim 1 to a cell culture.

9. The compound or use or method according to any one of claims 1 to 8 wherein said compound of formula (I) is TFA-12.
